# EUROPÄISCHE PATENTSCHRIFT

(11) **EP 2 413 854 B1**
(45) Veröffentlichungstag und Bekanntmachung des Hinweises auf die Patenterteilung: **13.03.2013**
(21) Anmeldenummer: 10712354.9
(22) Anmeldetag: 31.03.2010
(51) Int. Cl.: A61F 9/008, A61B 5/00

(54) **VORRICHTUNG ZUR NICHTINVASIVEN TEMPERATURBESTIMMUNG AN MIT EINER BEHANDLUNGSSTRAHLUNG BEHANDELTEM BIOLOGISCHEN GEWEBE**
DEVICE FOR NON-INVASIVE TEMPERATURE DETERMINATION IN BIOLOGICAL TISSUE TREATED WITH TREATMENT RADIATION
DISPOSITIF POUR LA DÉTERMINATION NON INVASIVE DE LA TEMPÉRATURE D'UN TISSU BIOLOGIQUE TRAITÉ PAR UN RAYONNEMENT DE TRAITEMENT

(30) Priorität: 03.04.2009 DE 102009016184
(43) Veröffentlichungstag der Anmeldung: 08.02.2012
(73) Patentinhaber: Carl Zeiss Meditec AG, 07745 Jena (DE)
(72) Erfinder: DICK, Manfred, 07926 Gefell (DE); DENNER, René, 99518 Reisdorf (DE); KNOKE, Stefan, 07745 Jena (DE)
(74) Vertreter: Beck, Bernard
(86) Internationale Anmeldenummer: PCT/EP2010/002048
(87) Internationale Veröffentlichungsnummer: WO 2010/112209

(56) Entgegenhaltungen:
- EP-A1- 1 279 385
- WO-A1-02/21646
- WO-A2-2009/056099
- DE-A1- 19 916 653
- DE-A1- 19 932 477

## Beschreibung

Die vorliegende Schrift beschreibt ein Verfahren zur nichtinvasiven Temperaturbestimmung an mit einer Behandlungsstrahlung behandeltem biologischen Gewebe, insbesondere an einem mittels Laserstrahlung behandelten Augenhintergrund, bei welchem mittels Messstrahlungspulsen eine von der Behandlungstemperatur abhängige Reaktion, insbesondere eine Druckwelle, erzeugt und mittels Detektoren erfasst wird. Die Erfindung betrifft eine Vorrichtung zur nichtinvasiven Temperaturbestimmung an mit einer Behandlungsstrahlung behandeltem biologischen Gewebe, insbesondere an einem mittels Laserstrahlung behandelten Augenhintergrund, mit einer Strahlungsquelle, einer Bestrahlungsoptik, Detektoren zur Bestimmung einer Reaktion, insbesondere einer Druckwelle, sowie einer zur Steuerung von Behandlungsstrahlung und Messstrahlungspulsen geeigneten Systemsteuerung.

### Stand der Technik

Der Einsatz von hochenergetischem Licht, beispielsweise gebündeltem Sonnenlicht, der Strahlung einer Xenon-Bogenlampe oder von Laserstrahlen in der Augenheilkunde, zum Beispiel zur Koagulation von retinalem Gewebe am Augenhintergrund, ist allgemein bekannt. Die Verwendung eines Laser als Strahlungsquelle hat dabei eine besondere Bedeutung erlangt, da sich einerseits die spektrale Wellenlänge des Laserlichts exakt festlegen lässt (monochromatisch, wahlweise definierte Wellenlängen im grünen, gelben, roten oder infraroten Spektralbereich)und andererseits eine präzise Lenkung des Laserlichts möglich ist.

In Abhängigkeit von dem verwendeten Lasertyp werden Laserpulse (z. B. beim Nd:YAG Laser, Wellenlänge 1064 nm, bzw. 532nm-frequenzverdoppelt) oder ein kontinuierlicher Laserstrahl (continuous wave = cw Laser, z. B. Argonlaser mit Wellenlängen von Blau = 488 nm und Grün = 514 nm, oder ebenfalls der frequenzverdoppelte Nd:YAG-Laser bei 532 nm) erzeugt. Argonlaser, Farbstoff- und Festkörper-Laser werden in der Regel zur Koagulation der Netzhaut bei diabetischer Retinopathie, bei peripherer Netzhautdegeneration, zur Behandlung von Netzhautlöchern sowie bei der Laser-Trabekuloplastik zur Verringerung des Augeninnendrucks eingesetzt. CO2-Laser dienen hingegen üblicherweise dem Durchtrennen von Gewebe.

Bei der thermischen Behandlung von Gewebe kommt der Temperaturführung eine besondere Bedeutung zu. Üblich sind bei der Laserphotokoagulation Bestrahlungszeiten von 20 ms bis 500 ms, insbesondere etwa 100 ms, zur Erzeugung von Temperaturen, die über 60 °C liegen. Die Laserleistung beträgt dabei in der Regel 100 mW bis 500 mW. Bei zu geringen Temperaturen ist der Koagulationseffekt unzureichend, bei zu hohen Temperaturen hingegen können unerwünschte Gewebeschädigungen auftreten.

Ein Verfahren und eine Vorrichtung der eingangs genannten Art, bei welchen eine Kontrolle der Behandlungstemperatur erfolgt, sind aus der Patentschrift DE 101 35 944 C2 bekannt. Die dort offenbarte Behandlungseinrichtung besteht aus einer Strahlungsquelle in Form eines kontinuierlich arbeitenden Lasers (cw-Laser), dessen Laserstrahl mittels einer Koppeloptik in einen Lichtleiter eingespeist und einer Bestrahlungsoptik und von dort dem zu behandelnden Auge zugeführt wird. Der Laserstrahl tritt dabei durch ein auf dem Auge aufsitzendes Kontaktglas hindurch, welches mit einem akustischen oder optischen Detektor ausgestattet ist.

Nach einem ersten Ausführungsbeispiel ist eine zusätzliche gepulste Strahlungsquelle vorgesehen, welche in vorgegebenen oder gesteuerten zeitabständen kurze Strahlungsimpulse erzeugt. Diese Lichtimpulse weisen eine hinsichtlich Pulsdauer und Energie von der Behandlungsstrahlung abweichende Charakteristik auf und werden über die Koppeloptik als Messstrahlung gleichfalls in den Lichtleiter eingespeist. Die Impulse der Messstrahlung führen im Auge zu einer thermischen Gewebeexpansion, welche von der vom Behandlungslaser verursachten Temperatur abhängig und mittels der zuvor genannten, beispielsweise piezoelektrischen Detektoren optoakustisch auswertbar ist. Die von der Messstrahlung hervorgerufene thermische Expansion ist dabei von der Temperatur linear abhängig, wobei die Abhängigkeit mittels einer Eichmessung bestimmt werden kann (Grüneisenkoeffizient).

Nach einem alternativen Vorschlag wird auf die zusätzliche Strahlungsquelle verzichtet und stattdessen der Behandlungsstrahl für einige Nanosekunden unterbrochen. Hierdurch wird eine Kontraktion des behandelten Gewebes am Augenhintergrund hervorgerufen, dessen Druckwelle ebenfalls zur Temperaturbestimmung herangezogen werden kann. Ein gesonderter Messstrahl ist in diesem Fall nicht vorgesehen.

Hinsichtlich des grundsätzlichen Aufbaus und der Verwendung von Laserstrahlung in der Augenheilkunde sowie von zur Erfassung der Expansion geeigneten Detektoren wird ausdrücklich auf die in der Patentschrift DE 101 35 944 C2 beschriebenen Ausführungsbeispiele verwiesen.

In der Offenlegungsschrift DE 199 16 653 A1 wird eine Vorrichtung zur individuellen Laserstrahlungsdosierung bei der transskleralen Laser-Zyklophotokoagulation offenbart, bei welcher im Zielgewebe der Behandlung Drucktransienten erzeugt werden, mittels derer vorab eine Behandlungsplanung erfolgt. Darüber hinaus können diese Informationen im weiteren Behandlungsverlauf zur Steuerung der Therapie genutzt werden. Dabei werden simultan zur Behandlungsstrahlung Diagnoseimpulse mit niedriger Energie erzeugt, welche dem Behandlungslaserpuls aufmoduliert oder vorweggeschickt werden. Die Messstrahlung kann dabei entweder von einer zusätzlichen Strahlungsquelle vorweggeschickt oder aber durch eine Modulation während der Koagulationsbestrahlung mit der Behandlungsstrahlungsquelle erzeugt werden.

Aus der Patentschrift DE 30 24 169 C2 sind ein weiteres Verfahren und eine weitere Vorrichtung zum Betreiben eines Photokoagulators für biologisches Gewebe insbesondere in der Augenheilkunde bekannt. Hierbei ist eine Einrichtung vorgesehen, welche den zeitlichen Verlauf der an der Koagulationsstelle herrschenden Leuchtdichte misst, welche durch die Behandlungsstrahlungsquelle oder einen von einer zusätzlichen Strahlungsquelle erzeugten Messstrahl hervorgerufen wird. Der zeitliche Verlauf der Leuchtdichte während der Strahlungsbehandlung wird dabei zur Regelung der Expositionsparameter herangezogen.

Die EP 1 279 385 lehrt eine Vorrichtung gemäß Oberbegriff des Anspruchs 1.

### Aufgabe

Der Erfindung liegt die Aufgabe zugrunde, den apparativen Aufwand zu verringern und dennoch eine besonders genaue Temperaturführung bei der Bestrahlungsbehandlung zu ermöglichen.

### Lösung

Die erfindungsgemäße Aufgabe wird durch die Merkmale des Anspruchs 1 gelöst. In Bezug auf eine Vorrichtung der eingangs genannten Art erfolgt die Lösung unter anderen dadurch, dass die Systemsteuerung zur Erzeugung von aufeinander folgenden Pulsen der Behandlungsstrahlungen und Messstrahlspulsen aus nur einer Strahlungsquelle, insbesondere einem modulierbaren cw-Laser, vorgesehen ist. Zusätzliche interne oder externe Modulatoren kommen dabei vorzugsweise nicht zum Einsatz.

Die Abfolge ist vorzugsweise alternierend ausgebildet, wobei auf genau einen Messstrahlungspuls genau ein Puls der Behandlungsstrahlung vorgesehen ist. Grundsätzlich ist es jedoch auch vorstellbar, zur Kontrolle des Messergebnisses zwei oder mehrere Messstrahlungspulse aufeinander folgen zu lassen oder den Puls der Behandlungsstrahlung zu unterbrechen, sofern im Verlauf der Behandlung wenigstens einem Messstrahlungspuls ein Puls der Behandlungsstrahlung und/oder wenigstens einem Puls der Behandlungsstrahlung ein Messstrahlungspuls folgt.

Durch die Erzeugung von Behandlungsstrahlung und Messstrahlspulsen mittels nur einer einzigen Strahlungsquelle wird der Bauaufwand ohne Verringerung der Messgenauigkeit reduziert. Darüber hinaus sind Behandlungsstrahlung und Messstrahlungspuls ohne weiteres Zutun auf dasselbe Zielvolumen im zu behandelnden Gewebe justiert.

Erfindungsgemäß werden Messstrahlungspulse und die Pulse der Behandlungsstrahlung von einem cw-Laser erzeugt, welcher durch eine entsprechende Modulation in besonderer Weise zur Erzeugung von schnell aufeinander folgenden Strahlungspulsen unterschiedlicher Charakteristik geeignet ist.

Der Messstrahlungspuls besitzt vorzugsweise eine Pulsenergie von 1 µJ bis 20 µJ, vorzugsweise größer 5 µJ, und/oder eine Pulslänge von 0,2 µs bis 2 µs. Hierdurch wird eine für eine genaue Messung besonders geeignete Druckwelle erzeugt, ohne dass der Messstrahlungspuls seinerseits signifikant zur Koagulation des Gewebes beiträgt. Mit besonderem Vorteil beträgt die Pulsspitzenleistung des Messstrahlungspulses dabei das 1,5 bis 5fache der Pulsleistung der Behandlungsstrahlung. Weiterhin ist eine Pulsfolge besonders günstig, bei welcher die Pulslänge des Behandlungsstrahles das 100fache bis 1000fache der Pulslänge des Messstrahlungspulses beträgt.

Mit Vorteil wird der Messstrahlungspuls mit einer Repetitionsrate von 500 Hz bis 10 kHz, vorzugsweise kleiner 1 kHz, erzeugt. Bei zu hohen Repetitionsraten würden sich die optisch erzeugten akustischen Wellen innerhalb des Auges und/oder des Detektors überlagern und zu einer Verfälschung oder zum Verlust von Primärinformation führen.

Ein besonders genaues Messergebnis wird erzielt, wenn die ansteigende Flanke des Messstrahlungspulses steiler als die ansteigende Flanke des Pulses des Behandlungsstrahles und/oder die abfallende Flanke des Messstrahlungspulses steiler als die abfallende Flanke des Pulses des Behandlungsstrahles eingestellt werden. Die Anstiegs- und/oder Abfallzeit des Messstrahlpulses beträgt besonders bevorzugt etwa das 0,01fache bis 0,1fache, insbesondere etwa das 0,05fache, der Anstiegs- und/oder Abfallzeit des Pulses der Behandlungsstrahlung. Ebenfalls ist bevorzugt vorgesehen, dass zwischen Messstrahlungspuls und dem darauf folgenden Puls der Behandlungsstrahlung eine Phase vollständiger oder insbesondere unvollständiger Leistungsreduzierung über einen Zeitraum von 0,5 µs bis 100 µs und/oder zwischen Puls der Behandlungsstrahlung und dem darauf folgenden Messstrahlungspuls eine Phase einer Leistungsreduzierung über einen Zeitraum von 50 µs bis 350 µs erfolgt.

Der in der genannten erfindungsgemäßen Vorrichtung vorgesehene modulierbare Laser weist mit Vorteil ein aktives Medium auf, welches die beim Pumpprozess zugeführte Energie für einen gewissen Zeitraum beispielsweise in Form einer Besetzungsinversion speichert und bei einem Anschwingen des Lichtfeldes im Laserresonator im Wesentlichen als Einzelimpuls / Erstimpuls abgibt. Ein geeignetes Medium weist beispielsweise Festkörperlaser auf, deren energetische Speicherzeit (auch Fluoreszenzlebensdauer oder Lebensdauer im oberen Laserniveau) üblicherweise im Bereich von 50 µs bis 1 ms liegt. Eine Besetzungsinversion liegt vor, wenn die Besetzung des energetisch höheren, am Laserverstärkungsprozess beteiligten oberen Laserniveaus größer ist als die des beteiligten unteren Laserniveaus.

Bevorzugt erfolgt die Modulation des Lasers in der Weise, dass zunächst eine Auszeit der Laserpumpquelle eingestellt wird, welche in etwa der genannten Speicherzeit des aktiven Lasermediums entspricht, um das vorhandene Strahlungsfeld im Laserresonator zu löschen und eine restliche Besetzungsinversion durch spontanen Zerfall abzubauen.

Durch ein schnelles Einschalten der Laserpumpquelle innerhalb von ca. 1-10µs wird infolge des mangelnden Strahlungsfeldes im Resonator zunächst ein überhöhter Aufbau der Inversion induziert, welche sich nach der dann folgenden Einstellung eines Strahlungsfeldes im Resonator (Anschwingvorgang) schlagartig wieder abbaut, wobei ein kurzer, intensiver Puls (Erstimpuls) entsteht, der Impulsdauern von etwa 1µs und Leistungsspitzenwerte von etwa 10 W (Pulsenergie 10µJ), bei einer maximalen cw-Laserleistung des Lasers von ca. 2 W, erreicht. Diese so erzeugten Pulse sind geeignet, ein auswertbares, optoakustisches Signal zu erzeugen.

Um insbesondere die fallende Flanke des Erstimpulses für die optoakustische Wirkung zu optimieren, wird bevorzugt die Laserpumpleistung anschließend kurzzeitig (z.B. für etwa 2µs) unter die danach folgende cw -Leistung geregelt.

Nach diesem Modulationszyklus bis höchstens zum Ende der durch die Repetition bestimmten Periodendauer wird die Laserpumpquelle kontinuierlich auf das für die Photokoagulation notwendige Niveau (z.B. für eine Laserausgangsleistung von 2W) geregelt, bis ein erneuter Modulationszyklus einsetzt. Diese Regelung erfolgt bevorzugt über eine Steuereinrichtung.

Bevorzugt werden dabei die Anstiegs- und Abfallzeiten des Koagulationsimpulses möglichst lange gewählt, damit diese keine zusätzliche detektierbare optoakustische Drucktransiente generieren. Bevorzugt werden dazu Zeiten im Bereich von 10µs - 50µs eingestellt.

Die periodische Ansteuerung des Lasers wird bevorzugt so lange fortgesetzt, bis das durch die erzeugte Photokoagulation bestimmte Abschaltkriterium erreicht ist und der Koagulationsprozess kontrolliert abgebrochen wird.

Zur Umsetzung der Erfindung besonders bevorzugt ist ein diodengepumpter Festkörperlaser, insbesondere ein Neodym-Yttrium-Vanadat-Festkörperlaser mit einer Wellenlänge von 1064 nm und Frequenzkonversion zur Wellenlänge 532 nm. Aufgrund der relativ kurzen Fluoreszenzlebensdauer des laseraktiven Mediums von 100 µs lässt sich die Ausschaltdauer bei der Modulation auf eine entsprechend geringe Zeitdauer begrenzen. Die Erzeugung und Steuerung der Strahlungspulse kann bei einem diodengepumpten Laser auf einfache Weise durch Modulation der Pumpquelle mittels geeigneter Beeinflussung des Diodenstroms durch die Systemsteuerung erfolgen.

Weiterhin kann mit Vorteil ein diodengepumpter Scheibenlaser, insbesondere ein Neodym-Yttrium-Vanadat-Scheibenlaser mit Frequenzverdopplung, zur Anwendung kommen. Durch die Ausbildung des Lasermediums als sehr dünne, in axialer Richtung gekühlte Scheibe wird der Aufbau einer thermischen Linse minimiert. Dies stellt insbesondere sicher, dass sich die Strahlungsparameter bei zeitlich variierender Ansteuerung während der Modulation und der Koagulationsdauer nicht verändern und somit der erfindungsgemäße Vorteil der Übereinstimmung von Mess- und Koagulationsvolumen in besonderer Weise zum Tragen kommt.

Weitere geeignete Strahlungsquellen können beispielsweise Diodenlaser, YAG-Laser oder diodengepumpte Halbleiterlaser sein. Weiterhin ist in nicht abschließender Aufzählung auch der Einsatz fokussierten Lichtes einer Xenonlampe, von Licht emittierenden Dioden (LED) oder Superlumineszenzdioden (SLD) möglich.

Grundsätzlich werden auch beispielsweise aus DE 10 2006 019 127 A1 bekannte Multiwellenlängen-Lasersysteme mit mehreren Strahlungsquellen von der beanspruchten Erfindung erfasst, sofern eine dieser Strahlungsquellen sowohl einen Messstrahlpuls als auch einen darauf folgenden Puls der Behandlungsstrahlung erzeugt. Die übrigen Strahlungsquellen werden dabei beispielsweise nur zusätzlich zur Koagulation verwendet und können die gleiche oder aber auch verschiedene Wellenlängen aufweisen.

### Figuren

Die Figuren stellen beispielhaft und schematisch eine Ausführung der Erfindung dar.

Es zeigen:
- Fig. 1: eine Prinzipskizze des Aufbaus einer Vorrichtung gemäß einer bevorzugten Ausführungsform der Erfindung;
- Fig. 2: ein Diagramm der Strahlungsleistung über die Zeit bei der Durchführung des beschriebenen Verfahrens.

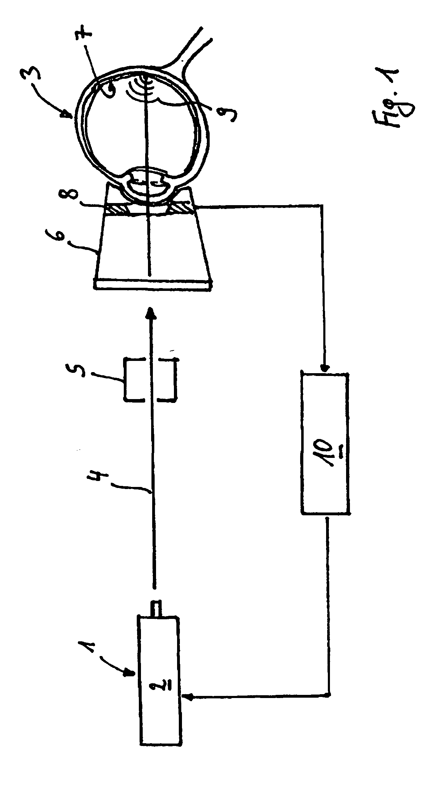

Die in Fig. 1 dargestellte Vorrichtung besteht aus einer Strahlungsquelle 1 in Form eines diodengepumpten cw-Lasers 2, welcher einen zur Behandlung eines Auges 3 vorgesehenen Laserstrahl 4 erzeugt. Der Laserstrahl 4 wird dem Auge 3 üblicherweise mittels eines nicht dargestellten Lichtleiters und einer Bestrahlungsoptik 5 durch ein Kontaktglas 6 hindurch zugeführt und trifft auf dem Augenhintergrund 7 auf.

Das Kontaktglas 6 ist mit einem Detektor 8 ausgestattet, der durch das Auftreffen des Laserstrahls 4 auf den Augenhintergrund 7 erzeugte Druckwellen 9 erfasst und die ermittelten Informationen an die Systemsteuerung 10 weiterleitet. Die Systemsteuerung 10 ist ihrerseits mit der Strahlungsquelle 1 wirkverbunden.

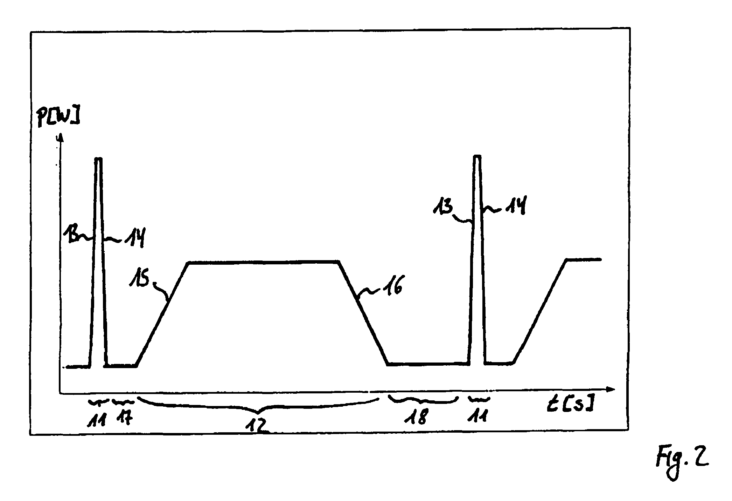

Bei Anwendung des beschriebenen Verfahrens zur nichtinvasiven Temperaturbestimmung an dem im Ausführungsbeispiel mit dem Laserstrahl 4 behandelten Augenhintergrund 7 werden von der an diesem Vorgang einzig beteiligten Strahlungsquelle 1, wie aus Fig. 2 ersichtlich, in alternierender Folge Messstrahlungspulse 11 und Pulse 12 der Behandlungsstrahlung erzeugt, welche am Augenhintergrund 7 dasselbe Zielvolumen treffen.

Jeder Messstrahlpuls 11 weist eine Impulsbreite von etwa 1 µs und eine Impulsspitzenleistung von rund 10 W bei einer Impulsenergie von etwa 10 µJ auf, wobei die mittlere Leistung des cw-Lasers nur rund 2 W betragen muss. Die ansteigende Flanke 13 und die abfallende Flanke 14 des Messstrahlpulses 11 verlaufen steil, so dass die Anstiegs- und/oder Abfallzeit des Messstrahlpulses 11 entsprechend kurz sind.

Die Anstiegs- und Abfallzeiten des auf den Messstrahlpuls 11 folgenden Pulses 12 des Behandlungsstrahles sind deutlich länger und betragen vorzugsweise 10 µs bis 50 µs. Ihre Dauer ist damit im Ausführungsbeispiel rund 20mal höher als Anstiegs- und/oder Abfallzeit des Messstrahlpulses 11, wodurch die ansteigende Flanke 15 und die abfallende Flanke 16 vergleichsweise flach verlaufen. Diese Ausbildung ist für den Koagulationsprozess besonders vorteilhaft. Die Leistung der Behandlungsstrahlung liegt im Ausführungsbeispiel bei 2 W, wobei der Puls 12 des Behandlungsstrahles über die Systemsteuerung 10 auf Wunsch zusätzlich unterbrochen werden kann. Zur Unterbrechung kann beispielsweise eine nicht dargestellte, von der Systemsteuerung angesteuerte Blende dienen.

Zwischen dem Messstrahlpuls 11 und dem darauf zeitlich folgenden Puls 12 der Behandlungsstrahlung ist eine Phase 17 reduzierter Laserleistung vorgesehen, in welcher der cw-Laser 2 aber weiterhin mit einer Restleistung (> 0 W) betrieben wird. Der Laserpumpstrom ist dabei so bemessen, dass er eine Stromstärke > 0 A aufweist, aber unterhalb der Laserschwelle des cw-Lasers 2 liegt. Eine weitere Phase 18 einer entsprechenden Reduzierung der Laserleistung wird über einen etwas längeren Zeitraum zwischen dem Puls 12 der Behandlungsstrahlung und dem darauf zeitlich folgenden Messstrahlpuls 11 eingeleitet.

Dieser Vorgang wiederholt sich vorzugsweise mit einer Repetitionsfrequenz von 1 kHz (Periodendauer 1000 µs). Jeder Modulationszyklus besteht dabei beispielsweise aus einer Abfolge von Verfahrensschritten folgender Dauer:

| | |
|---|---|
| 100 µs | Auszeit der Pumpquelle des cw-Lasers 2 als Löschzeit des Strahlungsfelds, führt zu Inversionszerfall; |
| 10 µs | Einschalten der Pumpquelle (Anschwingen des Messstrahlpulses 11); |
| 1 µs | Dauer des Messstrahlpulses 11; |
| 20 µs | Nachregelzeit (Phase 17) mit reduzierter Pumpleistung; |
| 869 µs | Dauer des Pulses 12 der Behandlungsstrahlung (Koagulationsdauer) mit einer kontinuierlichen Pumpleistung für eine Laser-Ausgangsleistung von 2 W und Anstiegs- und Anfallzeiten von je 50 µs. |

Die periodische Ansteuerung der Strahlungsquelle 1 wird fortgesetzt, bis das durch die Photokoagulation bestimmte Abschaltschaltkriterium erreicht ist und der Koagulationsprozess von der Systemsteuerung 10 kontrolliert abgebrochen wird. Die Dauer der Koagulationszeit verlängert sich gegenüber einer nicht erfindungsgemäß temperaturgeregelten Behandlung um rund 10%. Dieser zeitliche Mehraufwand wird jedoch durch die mit der Erfindung erzielten Vorteile überkompensiert.

### Bezugszeichenliste

- 1: Strahlungsquelle
- 2: cw-Laser
- 3: Auge
- 4: Laserstrahl
- 5: Bestrahlungsoptik
- 6: Kontaktglas
- 7: Augenhintergrund
- 8: Detektor
- 9: Druckwelle
- 10: Systemsteuerung
- 11: Messstrahlungspuls
- 12: Puls (der Behandlungsstrahlung)
- 13: Flanke (des Messstrahlungspulses, ansteigend)
- 14: Flanke (des Messstrahlungspulses, abfallend)
- 15: Flanke (Behandlungsstrahlpuls, ansteigend)
- 16: Flanke (Behandlungsstrahlpuls, abfallend)
- 17, 18: Phase (Strahlungsreduzierung)

- A: Ampere
- P: Leistung (der Strahlungsquelle)
- s: Sekunde
- t: Zeit
- W: Watt

## Patentansprüche

1. Vorrichtung zur nichtinvasiven Temperaturbestimmung an mit einer Behandlungsstrahlung behandeltem biologischen Gewebe, insbesondere an einem mittels Laserstrahlung behandelten Augenhintergrund (7), mit einer Strahlungsquelle (1), einer Bestrahlungsoptik (5), Detektoren (8) zur Bestimmung einer Reaktion, insbesondere einer Druckwelle, sowie einer zur Steuerung von Behandlungsstrahlung und Messstrahlungspulsen (11) geeigneten Systemsteuerung (10), **dadurch gekennzeichnet, dass** die Systemsteuerung (10) zur Erzeugung von aufeinander folgenden, insbesondere alternierenden Pulsen (12) von Behandlungsstrahlung und Messstrahlungspulsen (11) aus nur einer Strahlungsquelle (1) vorgesehen ist, wobei die Strahlungsquelle (1) ein modulierter cw-Laser (continuous wave laser 12) ist, der eine Erstimpulserzeugung in Kombination mit einer zeitlich dominanten cw-Laseremission nutzt.

2. Vorrichtung nach Anspruch 1, **dadurch gekennzeichnet, dass** der cw-Laser (12) ein vorzugsweise diodengepumpter Festkörperlaser ist.

3. Vorrichtung nach Anspruch 1, durch gekennzeichnet, dass der cw-Laser (12) ein diodengepumpter Scheibenlaser, insbesondere ein Neodym-Yttrium-Vanadat-Scheibenlaser, ist.

4. Vorrichtung nach Anspruch 1, durch gekennzeichnet, dass der cw-Laser (12) ein diodengepumpter Halbleiterlaser ist.

5. Vorrichtung nach Anspruch 1, durch gekennzeichnet, dass der cw-Laser (12) ein elektrisch gepumpter Halbleiterlaser ist.

6. Vorrichtung nach einem der vorhergehenden Ansprüche, **dadurch gekennzeichnet, dass** der Messstrahlungspuls (11) eine Pulsenergie von 1 µJ bis 20 µJ, vorzugsweise größer 5 µJ, und/oder eine Pulslänge von 0,2 µs bis 2 µs besitzt.

7. Vorrichtung nach einem der vorhergehenden Ansprüche, **dadurch gekennzeichnet, dass** die Pulsspitzenleistung des Messstrahlungspulses (11) das 1,5 bis 5fache der Spitzenleistung des Pulses (12) der Behandlungsstrahlung beträgt.

8. Vorrichtung nach einem der vorhergehenden Ansprüche, **dadurch gekennzeichnet, dass** die Länge des Pulses (12) des Behandlungsstrahles das 100fache bis 1000fache der Pulslänge des Messstrahlungspulses (11) beträgt.

9. Vorrichtung nach einem der vorhergehenden Ansprüche, **dadurch gekennzeichnet, dass** der Messstrahlungspuls (11) mit einer Repetitionsrate von 500 Hz bis 10 kHz, vorzugsweise kleiner 1 kHz, erzeugt wird.

10. Vorrichtung nach einem der vorhergehenden Ansprüche, **dadurch gekennzeichnet, dass** die ansteigende Flanke (13) des Messstrahlungspulses (11) steiler als die ansteigende Flanke (15) des Pulses (12) des Behandlungsstrahles eingestellt ist.

11. Vorrichtung nach einem der vorhergehenden Ansprüche, **dadurch gekennzeichnet, dass** die abfallende Flanke (14) des Messstrahlungspulses (11) steiler als die abfallende Flanke (16) des Pulses (12) des Behandlungsstrahles eingestellt ist.

12. Vorrichtung nach Anspruch 10 oder 11, **dadurch gekennzeichnet, dass** die Anstiegs- und/oder Abfallzeit des Messstrahlungspulses (11) etwa das 0,01fache bis 0,1fache, insbesondere etwa das 0,05fache, der Anstiegs- und/oder Abfallzeit des Pulses (12) der Behandlungsstrahlung beträgt.

13. Vorrichtung nach einem der vorhergehenden Ansprüche, **dadurch gekennzeichnet, dass** zwischen Messstrahlungspuls (11) und dem darauf folgenden Puls (12) der Behandlungsstrahlung eine Phase (17) einer Leistungsreduzierung über einen Zeitraum von 0,5 µs bis 100 µs erfolgt.

14. Vorrichtung nach einem der vorhergehenden Ansprüche, **dadurch gekennzeichnet, dass** zwischen Puls (12) der Behandlungsstrahlung und dem darauf folgenden Messstrahlungspuls (11) eine Phase (18) einer Leistungsreduzierung über einen Zeitraum von 50 µs bis 350 µs erfolgt.

## Claims

1. Device for noninvasively determining the temperature on biological tissue treated by means of treatment radiation, more particularly on a fundus (7) of the eye treated by means of laser radiation, comprising a radiation source (1), an irradiation optical unit (5), detectors (8) for determining a reaction, more particularly a shock wave, and a system control (10) suitable for controlling treatment radiation and measurement radiation pulses (11), **characterized in that** the system control (10) is provided for generating successive, more particularly alternating, pulses (12) of treatment radiation and measurement radiation pulses (11) from only one radiation source (1), the radiation source (1) being a modulated cw laser (continuous wave laser 12) which utilizes a first pulse generation in conjunction with a temporally dominant cw laser emission.

2. Device according to Claim 1, **characterized in that** the cw laser (12) is a preferably diode-pumped solid-state laser.

3. Device according to Claim 1, **characterized in that** the cw laser (12) is a diode-pumped disc laser, more particularly a neodymium-yttrium-vanadate disc laser.

4. Device according to Claim 1, **characterized in that** the cw laser (12) is a diode-pumped semiconductor laser.

5. Device according to Claim 1, **characterized in that** the cw laser (12) is an electrically pumped semiconductor laser.

6. Device according to one of the preceding claims, **characterized in that** the measurement radiation pulse (11) has a pulse energy from 1 µJ to 20 µJ, preferably greater than 5 µJ, and/or a pulse duration of 0.2 µs to 2 µs.

7. Device according to one of the preceding claims, **characterized in that** the pulse peak power of the measurement radiation pulse (11) is 1.5 to 5-times the peak power of the pulse (12) of the treatment radiation.

8. Device according to one of the preceding claims, **characterized in that** the duration of the pulse (12) of the treatment beam is 100-times to 1000-times the pulse duration of the measurement radiation pulse (11).

9. Device according to one of the preceding claims, **characterized in that** the measurement radiation pulse (11) is generated with a repetition rate of 500 Hz to 10 kHz, preferably of less than 1 kHz.

10. Device according to one of the preceding claims, **characterized in that** the rising flank (13) of the measurement radiation pulse (11) is set to be steeper than the rising flank (15) of the pulse (12) of the treatment beam.

11. Device according to one of the preceding claims, **characterized in that** the falling flank (14) of the measurement radiation pulse (11) is set to be steeper than the falling flank (16) of the pulse (12) of the treatment beam.

12. Device according to Claim 10 or 11, **characterized in that** the rise and/or decay time of the measurement radiation pulse (11) is approximately 0.01-times to 0.1-times, more particularly approximately 0.05-times, the rise and/or decay time of the pulse (12) of the treatment radiation.

13. Device according to one of the preceding claims, **characterized in that** there is a phase (17) of power reduction over a period of 0.5 µs to 100 µs between measurement radiation pulse (11) and the subsequent pulse (12) of the treatment radiation.

14. Device according to one of the preceding claims, **characterized in that** there is a phase (18) of power reduction over a period of 50 µs to 350 µs between a pulse (12) of the treatment radiation and the subsequent measurement radiation pulse (11).

## Revendications

1. Dispositif de détermination non invasive de la température sur un tissu biologique traité avec un rayonnement de traitement, notamment sur le fond d'un oeil (7) traité au moyen d'un rayonnement laser, comprenant une source de rayonnement (1), une optique de rayonnement (5), des détecteurs (8) pour déterminer une réaction, notamment une onde de pression, ainsi qu'une commande de système (10) adaptée pour commander le rayonnement de traitement et les impulsions de rayonnement de mesure (11), **caractérisé en ce que** la commande de système (10) est prévue pour générer des impulsions (12) successives, notamment alternées, de rayonnement de traitement et des impulsions de rayonnement de mesure (11) à partir d'une seule source de rayonnement (1), la source de rayonnement (1) étant un laser CW modulé (laser à onde continue 12) qui utilise la génération d'une première impulsion en combinaison avec une émission laser CW dominante dans le temps.

2. Dispositif selon la revendication 1, **caractérisé en ce que** le laser CW (12) est de préférence un laser à état solide à pompage par diode.

3. Dispositif selon la revendication 1, **caractérisé en ce que** le laser CW (12) est un laser à disque à pompage par diode, notamment un laser à disque au néodyme yttrium vanadate.

4. Dispositif selon la revendication 1, **caractérisé en ce que** le laser CW (12) est un laser à semiconducteur à pompage par diode.

5. Dispositif selon la revendication 1, **caractérisé en ce que** le laser CW (12) est un laser à semiconducteur à pompage électrique.

6. Dispositif selon l'une des revendications précédentes, **caractérisé en ce que** l'impulsion de rayonnement de mesure (11) possède une énergie d'impulsion de 1 µJ à 20 µJ, de préférence supérieure à 5 µJ, et/ou une longueur d'impulsion de 0,2 µs à 2 µs.

7. Dispositif selon l'une des revendications précédentes, **caractérisé en ce que** la puissance de crête d'impulsion de l'impulsion de rayonnement de mesure (11) est égale à 1,5 à 5 fois la puissance de crête de l'impulsion (12) du rayonnement de traitement.

8. Dispositif selon l'une des revendications précédentes, **caractérisé en ce que** la longueur de l'impulsion (12) du rayon de traitement est égale à 100 fois à 1000 fois la longueur d'impulsion de l'impulsion de rayonnement de mesure (11).

9. Dispositif selon l'une des revendications précédentes, **caractérisé en ce que** l'impulsion de rayonnement de mesure (11) est générée avec une fréquence de répétition de 500 Hz à 10 kHz, de préférence inférieure à 1 kHz.

10. Dispositif selon l'une des revendications précédentes, **caractérisé en ce que** le front montant (13) de l'impulsion de rayonnement de mesure (11) est réglé plus raide que le front montant (15) de l'impulsion (12) du rayon de traitement.

11. Dispositif selon l'une des revendications précédentes, **caractérisé en ce que** le front descendant (14) de l'impulsion de rayonnement de mesure (11) est réglé plus raide que le front descendant (16) de l'impulsion (12) du rayon de traitement.

12. Dispositif selon la revendication 10 ou 11, **caractérisé en ce que** le temps de montée et/ou de descente de l'impulsion de rayonnement de mesure (11) est égal à environ 0,01 fois à 0,1 fois, notamment à environ 0,05 fois, le temps de montée et/ou de descente de l'impulsion (12) du rayonnement de traitement.

13. Dispositif selon l'une des revendications précédentes, **caractérisé en ce qu'**une phase (17) de réduction de la puissance sur une période de 0,5 µs à 100 µs a lieu entre l'impulsion de rayonnement de mesure (11) et l'impulsion (12) qui suit du rayonnement de traitement.

14. Dispositif selon l'une des revendications précédentes, **caractérisé en ce qu'**une phase (18) de réduction de la puissance sur une période de 50 µs à 350 µs a lieu entre l'impulsion (12) du rayonnement de traitement et l'impulsion de rayonnement de mesure (11) qui suit.
